# EUROPEAN PATENT APPLICATION

(11) **EP 0 549 081 A1**
(43) Date of publication of application: **30.06.1993**
(21) Application number: 92204095.1
(22) Date of filing: 24.12.1992
(51) Int. Cl.: A61B 5/11, A61B 5/00, A61B 10/00

(54) **Device for measuring the activity of an animal**

(30) Priority: 24.12.1991 NL 9102182
(71) Applicant: STICHTING INSTITUUT VOOR MECHANISATIE ARBEID EN GEBOUWEN, NL-6700 AA Wageningen (NL)
(72) Inventor: Rossing, Wilhelm, NL-6708 KW Wageningen (NL); Te Brake, Josepheus HenricusAntonius, NL-3672 ET Driebergen (NL)
(74) Representative: de Bruijn, Leendert C.

(57) **Abstract**

The invention relates to a method for determining the condition of an animal. To this end, movements of the animal are recorded by a first sensor during a certain time interval and converted into electrical signals. Said signals are conveyed to an arithmetic unit and processed to give a characteristic value for that time interval. This characteristic value is compared with a reference value. The sensor can be an acceleration sensor which is fixed to the animal. By means of a second sensor, the temperature of the animal can be recorded. Processing of the signals to give a characteristic value, and storage of these signals and the characteristic value can advantageously take place on the animal itself.

## Description

The invention relates to a method for determining the condition of an animal, such as a pig, together with a device to be used with said method.

In the case of, for example, stock-raising and stock breeding, where a large number of animals is kept in one room, there are various advantages in being able to establish the condition of an individual animal in a simple manner. This is important, for example, in order to be able to recognise at an early stage that an animal is ill or otherwise deviates from the norm. In breeding it is, inter alia, also important to be able to establish the fertile periods of a female animal and the time when the animal is going to litter. Extensive research is therefore being carried out in this field at present, and a method is being sought to establish the condition of an animal, without human intervention, in a specific and reliable manner. In this context it is important that the animals themselves are bothered as little as possible by said method, while it should be possible to apply the method whenever required. The results moreover, should be available at virtually any time for each individual animal.

It is known that the condition of an animal can be determined on the basis of the body temperature of that animal. In the case of pigs, for example, the body temperature does not, however, depend on the condition of the animal, but also, inter alia, on the temperature of the surroundings, the stage of a sow in farrow, and even the time of day and the time when the animal has been fed. Large temperature fluctuations have been observed around the time of littering. In determining, for example, the fertile times of the animal, temperature measurements have been found to be less satisfactory. This also seems to be the case when detecting various illnesses (such as flu). Sensors, which were positioned relatively deeply inside the animal, in order to exclude environmental effects, were found to give better results in this respect, but these were still unreliable. Measured temperatures are usually transmitted continuously by means of telemetry to a central receiver and processing station.

There are various drawbacks associated with this known method. Thus, temperature measurements on their own can be used to determine only certain conditions of an animal. The reliability of this determination is questionable, however, since the temperature measurements may be considerably affected by the environment.

The object of the invention is to provide an improved method for determining the condition of an animal, which makes it possible to obtain results in a specific and reliable manner. To this end, during a certain time interval a first sensor records movements of an animal and converts them into electrical signals, which are conveyed to an arithmetic unit and are processed to give a characteristic value for that time interval. Said characteristic value is then compared with a reference value.

It has been found, namely, that the extent to which an animal moves and moves about, is strongly dependent on the condition of the animal. Recordings thereof surprisingly provided an effective measure for establishing the condition of an animal. In this way it is possible to establish a large number of conditions in a reliable and specific manner. With this method it is necessary to record the data regarding the movements during a certain time interval, in order to discount, for example, external deviations as far as possible.

For the purpose of measuring the movements of the animal advantageous use can be made of an acceleration sensor which detects both the size and the direction of the acceleration. The measured values can, for example, be plotted against time and then compared with a reference curve. Preferably, however, the signals are summed in the arithmetic unit, resulting in a single characteristic value or a number of easily comparable characteristic values.

In order to increase the accuracy, it is advantageous to measure not only the movements of the animal, but also the body temperature of the animal, with the aid of a second sensor. The values measured by the first and second sensor can be processed within a time interval independently of one another, to give characteristic values. The determination of the physical condition of an animal is improved, however, by these values being combined.

It may be expedient, prior to processing the signals to give a characteristic value, to suppress those signals whose value falls outside a certain range, for example interference signals. Depending on the measurement, however, only particular peak values may be of interest, which are summed over the time interval. In that case, all values below a certain threshold are disregarded for the purpose of the measurement.

For various reasons it is advantageous to store the signals, which have been measured by the first and/or the second sensor on the animal, at the transmitter assembly and to process them to give characteristic values, and then to transmit the latter, after the time interval has elapsed, to a central processing unit for further processing and readout. This minimises the energy consumption for, for example, measuring and telemetric transmission of the data. As a result, the necessary means to be fixed to the animal can be as small as possible, and the animal is therefore bothered as little as possible. Because of the low energy consumption, it is possible to operate, using the same battery, for a very long time. Since, in the case of pigs, it is definitely necessary to implant the measuring and transmission means, as there is a risk of these being eaten if they are attached to the skin, the invention makes it possible to implant a device according to the invention only once in the life of the animal.

In addition to comparing the characteristic value determined during the time interval with a reference value, it is advantageous to compare said value with a characteristic value of one or more previous time intervals, as well as with characteristic values of another animal, measured in the same or in a different time interval.

The length of a time interval can, for example, be determined by the time at which the animal is in the direct vicinity of a reception device. The time interval is terminated at that instant, and transmission takes place of the measured signals and/or the characteristic value determined. Because of the small distance between the transmitter and the receiver, transmission will require particularly little energy. The reception device could be located near the feeding trough, making it possible, if an animal recognition code is transmitted at the same time, to provide, for example, individual feed supplements. The information concerning the number of attempts an animal has made to take something, such as feed/water or feed salts, may also be of assistance in establishing the condition of the animal.

The invention will be discussed, on the basis of an illustrative, non-limiting embodiment, and by reference to the accompanying drawings, in which:
Fig. 1 shows the head of a pig in profile, and
Fig. 2 shows a schematic diagram of the layout of the device according to the invention, together with the central reception unit.

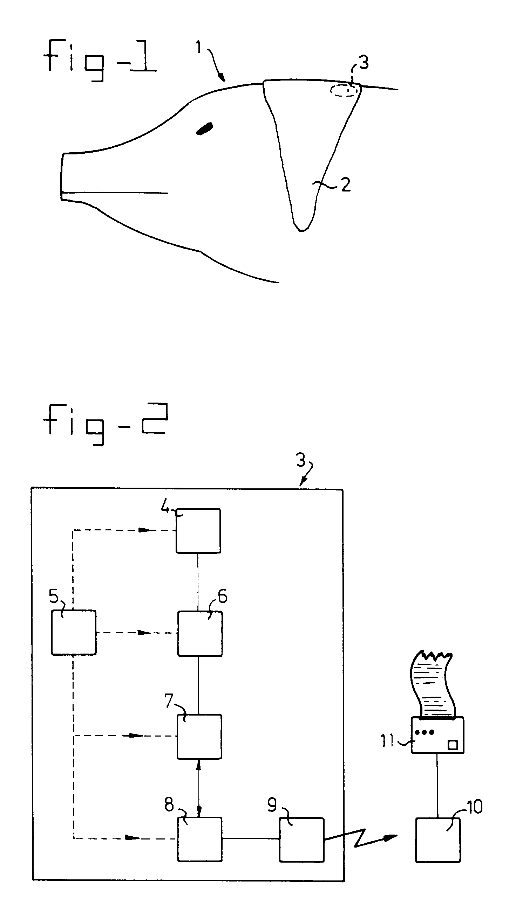

Fig. 1 shows the head of a pig 1 with a device 3 according to the invention implanted just below the skin close to the ears 2. Said device 3 is shown enlarged, for the sake of clarity, but in reality is of such a size that it can be fixed virtually painlessly with the aid of a hollow needle.

Fig. 2 shows, in diagrammatic form, the components contained in the device 3, as well as their mutual connections. A sensor 4 is shown, which can be used to measure the direction and magnitude of accelerations of the animal, and also to record the temperature of the animal. Said sensor 4 is supplied by a miniature NiCd battery. The sensor 4 converts the recorded movements and temperatures into electrical signals, separated according to direction, magnitude and type. Said signals are supplied to a filter 6, which suppresses signals having values outside a certain range. This filter 6, too, is supplied by means of the battery 5. The filtered signals coming from the filter 6 are passed on to an arithmetic unit 7 and are processed there to give one or more characteristic values. The arithmetic unit, however, can also send the signals to the memory 8 without processing them. In this way, said arithmetic unit 7 also exercises a control function. If the signals coming from the filter 6 are not separately stored in the memory, the arithmetic unit 7 will use them to determine one or more characteristic values and will store these in the memory. For example, the signals can be stored in the memory 8 until a certain number of signals has been collected, for example until the memory 8 has been filled to a particular level, whereupon the arithmetic unit uses said stored signals to determine a characteristic value and stores this in the memory 8, while the signals used are eliminated. In this way it is possible successively to determine, during a time interval, different characteristic values which can again be combined into one single characteristic value. At the end of the time interval the arithmetic unit 7, by means of the transmitter 9, sends the characteristic values and/or the signals coming from the filter 6 to a receiver 10. In this process, data can be extracted from the memory 8. The receiver 10 passes the received data to the central data processor 11. Said processor receives data from different animals. In the central data processor 11, a comparison could take place of the characteristic values with, for example, reference data or with characteristic values from other time intervals or from other animals. Said comparison can be printed out on paper, but it is also possible to provide a visual indication by means of indicators (not shown) located on the processing unit 11. For example, if a red indicator lamp lights up, this signifies that one of the animals from the group is in an abnormal condition. A green indicator lamp signifies that the condition is normal. Additionally, an audio signal can also be emitted.

Of course, the invention is not limited to the examples described above. It is defined more closely by the accompanying claims.

## Claims

1. Method for determining the condition of an animal, such as a pig, in which, during a certain time interval, a first sensor records movements of the animal and converts them into electrical signals, which are conveyed to an arithmetic unit and are processed to give a characteristic value for that time interval, which is then compared with a reference value.

2. Method according to Claim 1, in which the said first sensor is an acceleration sensor, which is fixed to the animal and which records accelerations of the animal and converts them into electrical signals.

3. Method according to Claim 2, in which said first sensor detects the direction of the acceleration and converts it into an electrical signal.

4. Method according to Claim 2 or 3, in which said first sensor detects the magnitude of the acceleration and converts it into an electrical signal.

5. Method according to one of the preceding claims, in which said electrical signals are summed in the arithmetic unit.

6. Method according to one of the preceding claims, in which said signals and/or the characteristic value are stored.

7. Method according to one of the preceding claims, in which during said time interval a second sensor records the temperature of the animal and converts it into an electrical signal, which provides a characteristic value for that time interval, which characteristic value is compared with a reference value.

8. Method according to Claim 7, in which electrical signals from the first and second sensors are processed together in an arithmetic unit to give a characteristic value for that time interval, which characteristic value is compared with a reference value.

9. Method according to one of the preceding claims, in which the electrical signals having a value outside a certain range are suppressed.

10. Method according to one of the preceding claims, in which the electrical signals from the first and/or second sensor on the animal are stored and are read out after the time interval has finished.

11. Method according to Claim 10, in which the signals stored on the animal, prior to readout, are processed in an arithmetic unit fixed to the animal.

12. Method according to one of the preceding Claims 10 or 11, in which the stored electrical signals and/or the characteristic values determined by means of the arithmetic unit are transmitted telemetrically, by means of a transmitter fixed to the animal, to a receiver for further processing.

13. Method according to one of the preceding claims, in which the end of the time interval is caused by the animal having come into close proximity to a receiver.

14. Method according to one of the preceding claims, in which the characteristic value or values from a time interval are compared with those of one or more previous time intervals.

15. Method according to one of the preceding claims, in which the characteristic value from a time interval is compared with the characteristic value of another animal, measured during the same or a different time interval.

16. Device for use in conjunction with the method according to one of the preceding claims, comprising a first sensor, which is suitable for recording movements and for converting said movements into electrical signals, a transmitter connected thereto and a current source for supplying said first sensor and said transmitter, which device is suitable for being moreover fixed under the skin of an animal.

17. Device according to Claim 16, moreover comprising a second sensor for recording a temperature and for converting the temperature into an electrical signal, which second sensor is connected to said current source.

18. Device according to Claim 16 or 17, further comprising a means for suppressing electrical signals from the first- and/or second sensor, if the value of those signals is outside a certain range.

19. Device according to Claim 16, 17 or 18, comprising an arithmetic unit which is connected to said first and, if appropriate, second sensor, and further comprising a storage medium connected to said arithmetic unit, said arithmetic unit and storage medium also being connected to the current source.

20. Device according to one of the preceding Claims 16 to 19 inclusive, comprising a means for emitting an animal recognition code.
